(19) **Europäisches Patentamt / European Patent Office / Office européen des brevets**

(11) **EP 2 889 363 B1**

(12) **EUROPEAN PATENT SPECIFICATION**

(45) Date of publication and mention
of the grant of the patent:
**01.03.2017 Bulletin 2017/09**

(51) Int Cl.:
***C12M 1/107*** *(2006.01)*   ***C12M 1/34*** *(2006.01)*
***C12M 1/36*** *(2006.01)*

(21) Application number: **13006020.5**

(22) Date of filing: **27.12.2013**

(54) **Optimal anaerobic environment maintenance system for producing biogas**

System zum Erhalten der optimalen anaeroben Umgebung zur Herstellung von Biogas

Système de maintien d'environnement anaérobie optimale pour produire du biogaz

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**01.07.2015 Bulletin 2015/27**

(73) Proprietor: **Liepajas Universitate
3401 Liepaja (LV)**

(72) Inventors:
• **Guseinovs, Sarifs**
  **LV-1067 Rïga (LV)**
• **Rimsans, Janis**
  **LV-1050 Rïga (LV)**
• **Aleksejeva, Jekaterina**
  **LV-1055 Rïga (LV)**

• **Junusovs, Sergejs**
  **LV-2010 Jürmala (LV)**
• **Jakimovs, Kirils**
  **LV-1029 Rïga (LV)**
• **Grickus, Armands**
  **LV-3401 Liepaja (LV)**
• **Andrejevs, Sergejs**
  **LV-1005 Rïga (LV)**
• **Zacs, Laimonis**
  **LV-3291 Talsu novads (LV)**

(56) References cited:
**EP-A1- 2 559 750       EP-A1- 2 586 856
CN-U- 201 574 152       US-A1- 2003 173 291
US-A1- 2011 048 722**

## Description

Technical field

The invention is related to biogas production and namely - to the systems anticipated for control and maintenance of anaerobic fermentation processes in the solid domestic waste layer.

Technical level

Chemical and bio-physical processes take place in the solid domestic waste layer, and as the result several gasses are emitted, which altogether are referred to as biogas. Biochemical processes depend on anaerobic bacteria. In usual conditions bacteria development and their life cycle are not regulated. For example, when temperature rises during warm days, the number of bacteria rapidly increases, and wastes are processed faster, and this, in its turn, increases the temperature of wastes and leads to death of bacteria colonies, therefore biogas is not anymore produced in this region even if there is a sufficient amount of unused material, from which biogas can be produced. This means that the environment can seriously influence the processes even in deeper solid domestic waste layers.

The document US 2003/0173291 A1 discloses a biogas production system with temperature, pressure and humidity sensors and programmable computer means for controlling the rate and extent of solid gasification. A specific placement system for the diverse sensors is not disclosed in this document.

Description of the invention

An automatic system is proposed, which allows maintaining the biogas producing anaerobic bacteria within the necessary temperature range that is, avoiding overheating, as well as providing the known special auxiliary substances and/or water from the previous technical level for provision of controlled watering. According to one manifestation of the invention the choice and location of sensor system are substantiated and this allows acquisition of better results by using the least number of sensors.

The proposed system contains humidity sensors; temperature sensors; pressure sensors; a data monitoring and analysis unit; a command unit; a system of water or auxiliary substance supply (watering), which contains the (underground) watering piping system and a liquid feeder; biogas collecting pipes. The humidity, temperature and pressure sensors are connected to the data monitoring and analysis unit, which has a possibility to send and receive electronic signals. The data monitoring and analysis unit, in its turn, is connected to the command unit and has a possibility to send and receive electronic signals. The command unit, in its turn, is connected to the liquid feeder and also has a possibility to send and receive electronic signals. The liquid feeder is designed with a possibility to selectively feed liquid (water and/or auxiliary substance) into the piping system for watering the corresponding segment of the solid household waste polygon. To provide the abovementioned function, the piping system is designed with a possibility to perform uniform watering at each segment of the solid household waste polygon.

The pressure sensors are installed with a possibility to perform data gathering for pressure inside the solid household waste polygon so that the sensor would not touch the solid or liquid components of the solid household waste polygon. The gas pressure is measured in the unconfined space, which is at least 125cm$^3$. The temperature sensors are installed with a possibility to perform data gathering about the temperature inside the solid household waste polygon, where the pressure is measured. In addition, sensors are installed in such a way that they do not increase the measurement error if placed in the acidic or saline environment. The advisable temperature range for the sensor is from -10 to +100 °C. The humidity sensors are made and located with a possibility to measure relative humidity in respect to the dew point. Moreover, the sensor is installed in such a way that it would not increase the measurement error if placed in the acidic or saline environment. The advisable measurement range for humidity is from 0% to 100%.

Each sensor is installed according to the topology and morphology of the studied area. The offered system anticipates formation of equal area segments at the biogas producing solid household waste polygon, and also placement of a group of sensors in each segment, where humidity sensors are placed at the borders of each segment, but the temperature and pressure sensors are placed at the centre of each segment. During the research it was found out that using only humidity sensors is not efficient because in this case it would be necessary to have a large amount of such sensors. It turned out that if pressure and temperature sensors are used as well, then the total number of sensors needed can be significantly decreased. In addition, pressure and temperature sensors should be placed next to each other, in order to measure pressure and temperature simultaneously at one point.

The data monitoring and analysis unit accumulates data, which are received from the sensors. The data monitoring and analysis unit identifies the sensor, from which the information is read, by using a system of relays, where each sensor has a number, which is transformed to the binary system, for example, the sensor with the number 24 will be 10110, and at the corresponding levels (the first, the third and the fourth) the relay is switched on and the signal is obtained from the needed sensor. After transforming data into digital format, they are recorded in the memory unit. The memory unit contains the last values of sensors, which have not been read yet. The data monitoring and analysis unit, when activating the certain sensor with the help of the system, waits for some time and tries to read data, then after the successful read it performs the procedure again several times within a few seconds, in order to acquire an averaged value from the obtained results. After acquisition of a time stamp and reading values of the certain sensor (the values range from 4 to 20 mA), the data monitoring and analysis unit transforms these values to Celsius degrees, bars and humidity percentage. This is done by using a predefined sensor map, where each sensor has its own values, address and wire resistance compensation parameters. Thus, when acquiring a value of 11.312 mA, a wire compensation for the initial value is applied, which is for instance, +1.621. This is needed, because the wire has resistance and the value is read by taking into account the length of the wire and the specific electrical resistance. In addition, the mentioned sensor map contains the information about the type of the sensor used (it allows definition of converting parameters - to which value it is necessary to convert), for example, it can be a temperature sensor. The conversion is done by taking into account the lower threshold of 4 mA and the higher threshold of 20 mA, and this means that if the sensor temperature changes within the range from 0 to 100 degrees, the sensor current changes from 4 to 20 mA, and the change is proportional, therefore, by knowing the values of current, it is possible to calculate the temperature of the sensor. Such values are fixed in the sensor value map and in the software.

Acquiring the corresponding signal from the data monitoring and analysis unit, the command unit activates a valve, which opens a group of watering pipes, which perform watering of a certain segment of the polygon. Valves have a piezoelectric mechanism; this means that the liquid flow is opened only when there is a voltage supplied. In order to avoid penetration of into the water and auxiliary substance supply system, a safety valve is uses, which does not allow the gas going through the watering system.

In the result of short-term research the inventors elaborated the optimal scheme for placing the sensors in the proposed system, where the coordinates of the sensors are obtained according to the formulae described below, where the reference point is the south-eastern corner of the solid household waste polygon.

Determination of positions of humidity sensors

$$S_h^{i,j} = \left( \underbrace{\frac{2-\sqrt{3}}{\sqrt{160}} \cdot p_j^{length} + (i-1) \cdot \left( \frac{p_j^{length}}{3} - \frac{2}{3} \cdot \frac{2-\sqrt{3}}{\sqrt{160}} \right) \cdot p_j^{length}}_{x}, \underbrace{p_j}_{y}, \underbrace{p_j^{height} + d_j}_{z} \right), i = \overline{1,3}; j = \overline{1,n}_{pipes};$$

Determination of positions of temperature and pressure sensors

$$S_{tp}^{i,j} = \left( \underbrace{\frac{p_j^{length}}{6} + \frac{4-2\cdot\sqrt{3}}{3\cdot\sqrt{160}} p_j^{length} + (i-1) \cdot \left( \frac{p_j^{length}}{3} - \frac{2}{3} \cdot \frac{2-\sqrt{3}}{\sqrt{160}} \right) \cdot p_j^{length}}_{x}, \underbrace{p_j}_{y}, \underbrace{p_j^{height}}_{z} \right), i = 1,2; j = \overline{1,n}_{pipes};$$

where

$S_h^{i,j}$ is the position of the i-th humidity sensor, which is located next to the j-th gas collecting pipe;

where $S_{tp}^{i,j}$ is the position of the i-th temperature and pressure sensors, which are located next to the j-th gas collection pipe;

$n_{pipes}$ is the quantity of the collected biogas, which is prone to change in respect to the placement map, and can change from 3 to 5;

$p_j^{length}$ - is the length of the j-th gas collection pipe;

$p_j^{height}$ - is the height of the j-th gas collection pipe from the ground level;

$d_j$ - is the diameter of the j-th gas collection pipe;

i - is the number of each sensor;

j - is the number of each pipe, showing if it is the first, the second or the third pipe;

$p_j$ - is the distance between the higher pipe wall and the surface of the SHW polygon, that is, it is the depth of the pipe (the depth till the higher part of the pipe);

x,y,z - are the coordinates in the 3D space, x showing the coordinate in the direction of the polygon length, y - in the direction of the polygon width (the same as for pipes) and z is the depth in respect to the gas collection pipe.

For example, (2,1,0.15) denotes that the sensor is placed 2 metres away in the direction along the pipe at the first pipe and 0.15 m above it (if 0.15 m is the diameter of the pipe).

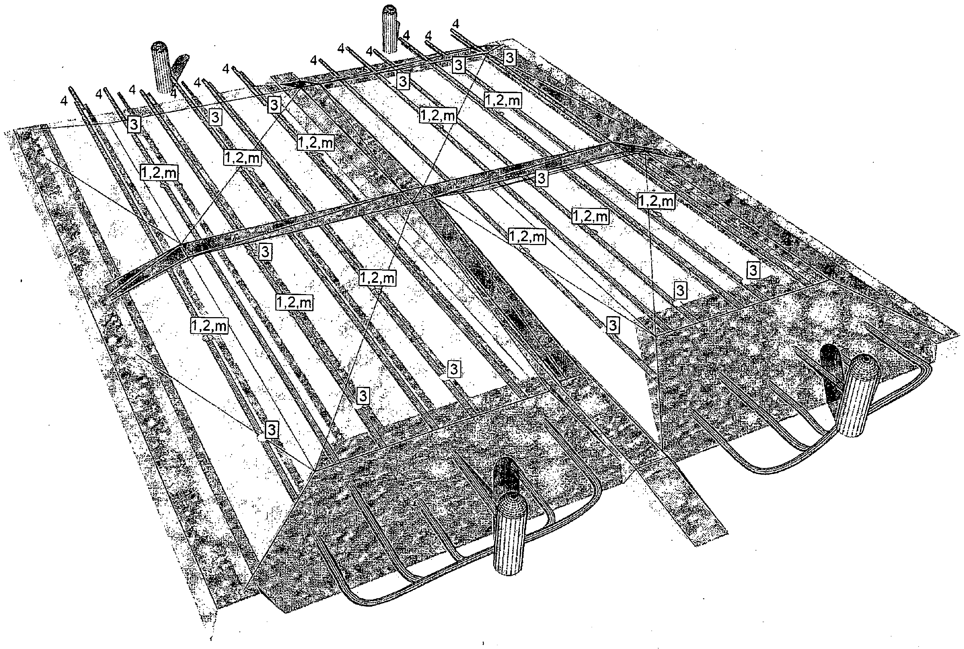

An example of implementation of the invention is shown in Fig. 1, where a 3D model of the solid household waste polygon is shown, which is placed according to the proposed invention, where M are the damping points of the watering system, 1 - temperature sensors, 2 - pressure sensors and 3 - humidity sensors, 4 - gas collection pipes.

**Claims**

1. A system for maintenance of optimal anaerobic environment in biogas production, which contains: humidity, pressure and temperature sensors placed in the layer of the solid household wastes; the data monitoring and analysis unit; the command unit; the water and auxiliary substance supply system, which contains the liquid feeder and the system of pipes, located in the layer of solid household wastes; the humidity, pressure and temperature sensors are connected to the data monitoring and analysis unit having a possibility to send and receive electronic signals, which is connected to the command unit having also a possibility to send and receive electronic signals, which, in its turn, is connected to the liquid feeder having a possibility to send and receive electronic signals; the liquid feeder is designed with a possibility, when receiving a command from the command unit, to selectively supply liquid into the system of watering pipes for watering a segment of the solid household waste polygon, whereas the watering pipe system is designed with a possibility to provide uniform watering of a segment of the solid household waste polygon, where each mentioned segment of the waste polygon contains humidity sensors, which are placed at the borders of the segment, at least one temperature sensor and at least one pressure sensor, which are placed in the centre of the segment; the position of the humidity sensor is chosen in such a way that the coordinates (x, y, z) of the humidity, pressure and temperature sensors would correspond to the existing formulas, where the reference point is the south-eastern corner of the solid household waste polygon:

determination of humidity sensor positions -

$$S_h^{i,j} = \left( \underbrace{\frac{2-\sqrt{3}}{\sqrt{160}} \cdot p_j^{length} + (i-1) \cdot \left( \frac{p_j^{length}}{3} - \frac{2}{3} \cdot \frac{2-\sqrt{3}}{\sqrt{160}} \right) \cdot p_j^{length}}_{x}, \underbrace{p_j}_{y}, \underbrace{p_j^{height} + d_j}_{z} \right), i = \overline{1,3}; j = \overline{1,n}_{pipes};$$

determination of pressure and temperature sensor position -

$$S_{tp}^{i,j} = \left( \underbrace{\frac{p_j^{length}}{6} + \frac{4-2\cdot\sqrt{3}}{3\cdot\sqrt{160}} p_j^{length} + (i-1) \cdot \left( \frac{p_j^{length}}{3} - \frac{2}{3} \cdot \frac{2-\sqrt{3}}{\sqrt{160}} \right) \cdot p_j^{length}}_{x}, \underbrace{p_j}_{y}, \underbrace{p_j^{height}}_{z} \right), i = 1, 2; j = \overline{1,n}_{pipes};$$

where, $S_h^{i,j}$ is the position of the i-th humidity sensor, which is located next to the j-th gas collecting pipe;

where, $S_{tp}^{i,j}$ is the position of the i-th temperature and pressure sensors, which are located next to the j-th gas collection pipe; $n_{pipes}$ is the quantity of the collected biogas, which is prone to change in respect to the placement map, and can change from 3 to 5; $p_j^{length}$ is the length of the j-th gas collection pipe; $p_j^{height}$ is the height of the j-th gas collection pipe, from the ground level;

$d_j$ - is the diameter of the j-th gas collection pipe; i - is the number of each sensor;

j - is the number of each pipe, showing if it is the first, the second or the third pipe; $p_j$ is the distance between the higher pipe wall and the surface of the SHW polygon, that is, it is the depth of the pipe (the depth till the higher part of the pipe); x,y,z - are the coordinates in the 3D space, x showing the coordinate in the direction of the polygon length, y - in the direction of the polygon width (the same as for pipes) and z is the depth in respect to the gas collection pipe.

## Patentansprüche

1. Ein System zur Aufrechterhaltung einer optimalen anaeroben Umgebung in der Biogasproroduktion, die enthält: Feuchtigkeits-, Druck- und Temperatursensoren in der Schicht der festen Haushaltsabfälle; die Datenüberwachungs- und Analyseeinheit; die Befehlseinheit; das Wasser- und Hilfsstoffversorgungssystem, das die Flüssigkeitszuführung und das Rohrsystcm enthält und sich in der Schicht von festen Haushaltsabfällen befindet; die Feuchte-, Druck- und Temperatursensoren sind mit der Datenüberwachungs- und Analyseeinheit verbunden, welche die Möglichkeit hat elektronische Signale zu senden und zu empfangen und ist mit der Befehlseinheit verbunden, welche auch die Möglichkeit hat elektronische Signale zu senden und zu empfang, und welche ist seinerseits mit dem Flüssigkeitszuführer verbunden, der die Möglichkeit hat, elektronische Signale zu senden und zu empfangen; die Flüssigkeitszuführer ist so ausgestaltet, dass sie bei Empfang eines Befehls von der Befehlseinheit selektiv Flüssigkeit in das System von Bewässerungsrohren zum Bewässern eines Abschnitts des Haushalts-Feststoffabfall Polygons zuführen kann, während das Bewässerungsrohrsystem mit der Möglichkeit entworfen ist Ein gleichmäßiges Bewässern eines Abschnitts des Haushalts-Fcststoffabfall Polygons, wo jedes genannte Segment des Abfallpolygons enthält an den Rändern des Segments angeordnete Feuchtesensoren, mindestens einen Temperatursensor und mindestens einen Drucksensor, die angeordnet sind In der Mitte des Segments; die Position des Feuchtesensors ist so gewählt, dass die Koordinaten ($x,y,z$) der Feuchte-, Druck- und Temperatursensoren den vorhandenen Formeln entsprechen, wobei der Bezugspunkt ist die südöstliche Ecke des Haushalts-Feststoffabfall Polygons: Bestimmung von Feuchtesensorpositionen -

$$S_h^{i,j} = \left( \underbrace{\frac{2-\sqrt{3}}{\sqrt{:60}} \cdot p_j^{length} + (i-1) \cdot \left( \frac{p_j^{length}}{3} - \frac{2}{3} \cdot \frac{2-\sqrt{3}}{\sqrt{:60}} \right) \cdot p_j^{length}}_{x}, \underbrace{p_j}_{y}, \underbrace{p_j^{height} + d_j}_{z} \right), i = \overline{1,3}; j = \overline{1, n}_{pipes};$$

Bestimmung der Druck- und Temperatursensorposition -

$$S_{tp}^{i,j} = \left( \underbrace{\frac{p_j^{length}}{6} + \frac{4-2\cdot\sqrt{3}}{3\cdot\sqrt{160}} p_j^{length} + (i-1) \cdot \left( \frac{p_j^{length}}{3} - \frac{2}{3} \cdot \frac{2-\sqrt{3}}{\sqrt{160}} \right) \cdot p_j^{length}}_{x}, \underbrace{p_j}_{y}, \underbrace{p_j^{height}}_{z} \right), i = 1,2; j = \overline{1, n}_{pipes};$$

wobei $S_h^{i,j}$ die Position des *i-ten* Feuchtigkeitssensors ist, die sich neben dem j-*ten* Gassammelrohr befindet; wobei $S_{tp}^{i,j}$ die Position der *i-ten* Temperatur- und Drucksensoren ist, die sich neben dem j-*ten* Gassammelrohr befnden; $n_{pipes}$ ist die Menge des gesammelten Biogases, die anfällig für Veränderungen ist in Bezug auf die Platzierungskarte und kann von 3 bis 5 ändern; $p_j^{length}$ die Länge des j-*ten* Gassammelrohrs ist; $p_j^{height}$ die Höhe des j-*ten* Gassam- melrohrs vom Bodenniveau ist; $d_j$ der Durchmesser des j-ten Gassammelrohres ist; i - die Nummer jedes Sensors ist; j - die Nummer jedes Rohres ist, was zeigt ob es das erste, das zweite oder das dritte Rohr ist; $p_j$ - der Abstand zwischen der höheren Rohrwand und der Oberfläche des Haushalts-Feststoffabfall Polygons ist, das heißt, es ist die Tiefe des Rohres (die Tiefe bis zum oberen Teil des Rohres); $x,y,z$ sind die Koordinaten im 3D-Raum, wobei $x$ die Koordinate in Richtung der Polygonlänge und $y$ - die Koordinate in Richtung der Polygonbreite (gleich wie bei Rohren) ist, und $z$ ist die von dem Gassammelrohr gemessene Tiefe.

**Revendications**

1. un système de maintien d'environnement anaérobie optimal pour produire du biogaz, qui contient: les capteurs d'humidité, pression et température, qui sont places dans la couche des déchets solides domestiques; l'unité de registration et d'analyse des données; l'unité de commande; le système de la provision d'eau et de substances auxiliaires, qui contiennent un fournisseur des liquides et un système des tuyaux, qui sont placées dans la couche solide des déchets domestiques. Les capteurs d'humidité, pression et température sont lies avec l'unité de registration et d'analyse des données, donc il y a la possibilité d'envoyer et recevoir des signaux électroniques. L'unité de registration et d'analyse des données est liée avec le fournisseur des liquides, qui a aussi la possibilité d'envoyer et recevoir des signaux. Le fournisseur des liquides est construit avec la possibilité, que, le moment il reçoit une commande d'unité de commande, il peut fournir liquide au système des tuyaux d'arrosage d'un segment de couche solide sélectivement. Le système des tuyaux d'arrosage est construit avec la possibilité de fournir arrosage uniforme d'un segment de polygone des déchets solides domestiques, ou chaque segment de polygone des déchets mentionné contient les capteurs d'humidité, qui sont placés dans des points de frontières du segment; au minimum un capteur de température et au minimum un capteur de pressure, qui sont placés dans le centre du segment. La position de capteur d'humidité est choisie dans la manière, ou les coordonnées ($x,y,z$) de capteurs d'humidité, pressure et température sont correspondantes aux formules existantes, ou le point de référence est le coin sud-est du polygone des déchets domestiques solides:

détermination des positions des capteurs d'humidité -

$$S_h^{i,j} = \left( \underbrace{\frac{2-\sqrt{3}}{\sqrt{160}} \cdot p_j^{length} + (i-1)\cdot\left(\frac{p_j^{length}}{3} - \frac{2}{3}\cdot\frac{2-\sqrt{3}}{\sqrt{160}}\right)\cdot p_j^{length}}_{x}, \underbrace{p_j}_{y}, \underbrace{p_j^{height}+d_j}_{z} \right), i=\overline{1,3}; j=\overline{1,n}_{pipes};$$

détermination de la position du capteur de pressure et température -

$$S_{tp}^{i,j} = \left( \underbrace{\frac{p_j^{length}}{6} + \frac{4-2\cdot\sqrt{3}}{3\cdot\sqrt{160}} p_j^{length} + (i-1)\cdot\left(\frac{p_j^{length}}{3} - \frac{2}{3}\cdot\frac{2-\sqrt{3}}{\sqrt{160}}\right)\cdot p_j^{length}}_{x}, \underbrace{p_j}_{y}, \underbrace{p_j^{height}}_{z} \right), i=1,2; j=\overline{1,n}_{pipes};$$

ou $S_h^{i,j}$ est la position de *i-éme* capteur d'humidité, qui est place à côté de *j-ème* tuyau de collection du gaz;

ou $S_{tp}^{i,j}$ est la position de *i-ème* capteurs de température et pressure, qui sont places à côté de *j-ème* tuyau de collection du gaz; $n_{pipes}$ est la quantité de biogaz collecte, qui est sujette à changer en accord avec le placement, donc elle peut changer de 3 à 5; $p_j^{length}$ est la longueur de *j-ème* tuyau de collection du gaz; $p_j^{height}$ est la hauteur de *j-ème* tuyau de collection du gaz, du niveau du sol;

$d_j$ est le diamètre de *j-ème* tuyau de collection du gaz; $i$ est le numéro de chaque capteur;
$j$ est le numéro de chaque tuyau, qui expose, est-ce que c'est le premier, deuxième ou troisième tuyau; $p_j$ est la distance entre le tuyau le plus haut et la paroi de tuyau supérieure et la surface du polygone des déchets domestiques solides, donc, c'est la profondeur de tuyau (la profondeur jusqu'à la partie supérieure du tuyau); $x,y,z$ sont les coordonnées dans l'espace 3D, $x$ expose la coordonnée dans la direction de longueur du polygone, $y$ expose la coordonnée dans la direction de largeur du polygone (la même pour des tuyaux) et $z$ est la profondeur à tuyau de collection du gaz.

**EP 2 889 363 B1**

**Patent documents cited in the description**

- US 20030173291 A1